# FASCICULE DE BREVET EUROPEEN

(11) **EP 3 801 066 B1**
(45) Date de publication et mention de la délivrance du brevet: **30.08.2023**
(21) Numéro de dépôt: 19740620.0
(22) Date de dépôt: 22.05.2019
(51) Int. Cl.: A23L 33/155, A23K 20/174, A01K 67/033, A23J 1/00, A23K 10/20, A23K 50/90

(54) **PROCEDE D'ELEVAGE DE COLEOPTERES COMPRENANT UN TRAITEMENT ULTRAVIOLET POUR LA PREPARATION D'UNE POUDRE DE COLEOPTERES ENRICHIE EN VITAMINE D3**
ERFAHREN ZUM ZÜCHTEN VON KÄFERN MIT EINER UV-BEHANDLUNG ZUR HERSTELLUNG VON MIT VITAMIN D3 ANGEREICHERTEM KÄFERPULVERS
METHOD FOR RAISING BEETLES COMPRISING AN ULTRAVIOLET TREATMENT FOR PREPARING A BEETLE POWDER ENRICHED WITH VITAMIN D3

(30) Priorité: 28.05.2018 FR 1854534
(43) Date de publication de la demande: 14.04.2021
(73) Titulaire: Nutri'Earth, 59160 Lille (FR)
(72) Inventeur: DEFRIZE, Jerémy, 59320 Santes (FR); DORMIGNY, Thomas, 62410 Meurchin (FR); DESTAILLEUR, Charles-Antoine, 59320 Emmerin (FR)
(74) Mandataire: RVDB
(86) Numéro de dépôt international: PCT/FR2019/051168
(87) Numéro de publication internationale: WO 2019/229332

(56) Documents cités:
- WO-A1-2016/108037
- M.D. FINKE: "Complete nutrient content of three species of wild caught insects, pallid-winged grasshopper, rhinoceros beetles and white-lined sphinx moth", JOURNAL OF INSECTS AS FOOD AND FEED, vol. 1, no. 4, 7 décembre 2015 (2015-12-07), pages 281-292, XP055540394, DOI: 10.3920/JIFF2015.0033
- Wit: "Rearing of Bombyx mori with a vitamin D enriched diet", , juin 2017 (2017-06), XP055540370, Almere Extrait de l'Internet: URL:https://www.greeni.nl/webopac/MetaData EditDownload.csp?file=2:143041:1 [extrait le 2019-01-10]
- BIRGIT A. RUMPOLD ET AL: "Nutritional composition and safety aspects of edible insects", MOLECULAR NUTRITION & FOOD RESEARCH, vol. 57, no. 5, mai 2013 (2013-05), pages 802-823, XP055540396, DE ISSN: 1613-4125, DOI: 10.1002/mnfr.201200735
- MARK D. FINKE ET AL: "Complete nutrient content of four species of commercially available feeder insects fed enhanced diets during growth : Complete Nutrient Content of Four Species of Feeder Insects", ZOO BIOLOGY, vol. 34, no. 6, novembre 2015 (2015-11), pages 554-564, XP055540382, US ISSN: 0733-3188, DOI: 10.1002/zoo.21246
- LENKA KOURIMSKÁ ET AL: "Nutritional and sensory quality of edible insects", NFS JOURNAL, vol. 4, octobre 2016 (2016-10), pages 22-26, XP055540649, ISSN: 2352-3646, DOI: 10.1016/j.nfs.2016.07.001
- MASATOSHI HORI ET AL: "Lethal effects of short-wavelength visible light on insects", SCIENTIFIC REPORTS, vol. 4, no. 1, 9 décembre 2014 (2014-12-09), XP055540419, DOI: 10.1038/srep07383
- FINKE MARK D: "Gut loading to enhance the nutrient content of insects as food for reptiles: A mathematical approach", ZOO BIOLOGY, JOHN WILEY & SONS, INC., HOBOKEN, NJ, US, vol. 22, no. 2, 2003, pages 147-162, XP002786852, ISSN: 0733-3188
- Mark D. Finke ET AL: "Insects as Food for Insectivores" In: "Mass Production of Beneficial Organisms", 2014, Amsterdam [u.a.] : Acad. Press/Elsevier, c 2014, NL, XP055525928, ISBN: 978-0-12-391453-8 pages 583-616, DOI: 10.1016/B978-0-12-391453-8.00017-0, page 594
- Anonymous: "Coleoptera - Wikipédia", , 3 mai 2018 (2018-05-03), XP055540766, Extrait de l'Internet: URL:https://fr.wikipedia.org/w/index.php?t itle=Coleoptera&oldid=148109108 [extrait le 2019-01-11]

## Description

### Domaine technique et art antérieur

La présente invention concerne le domaine de l'alimentation.

L' objet de la présente invention vise un procédé d"élevage des coléoptères pour la préparation d'une poudre de coléoptères enrichie en vitamine D3

Aujourd'hui, on recense plus de deux milles espèces d'insectes comestibles dans le monde (d'après une source de la FAO).

L'utilisation d'insectes comestibles en tant que source nutritive pour l'alimentation humaine ou animale représente un potentiel non-exploité qui est de plus en plus reconnu, notamment en Europe.

Des études ont montré que certaines espèces d'insectes allient des propriétés nutritionnelles très intéressantes aussi bien sur le plan qualitatif que quantitatif.

On sait par ailleurs que la production d'aliments à base d'insectes a un impact réduit sur l'environnement (voir notamment les publications *Belluco et al., 2013, Oonincx et al, 2010* ou encore *Van Huis et al. 2013*).

Des études sur le pouvoir nutritionnel de certaines espèces de coléoptères ont été approfondies ces dernières années.

Chez les espèces *Tenebrio molitor* ou *Alphitobius diaperinus,* ce sont les stades larvaires et le stade nymphal qui sont les plus intéressants d'un point de vue nutritionnel.

Ces études montrent en effet que les larves et les nymphes de ces deux espèces contiennent une grande diversité de nutriments d'intérêts parmi lesquels des protéines (entre 50 et 66% du poids sec), des acides gras essentiels comme les oméga 3 dont des ALA, des vitamines ou encore des minéraux.

Parmi les nutriments d'intérêts présents chez ces coléoptères, on retrouve plus particulièrement la vitamine D3 (ou cholécalciférol) qui est une forme de la vitamine D.

La vitamine D3 a des propriétés importantes.

Chez l'espèce humaine, la vitamine D3 participe au maintien des niveaux sanguins normaux de calcium et de phosphore absorbés par l'intestin.

La vitamine D3 joue donc un rôle essentiel dans le maintien des muscles squelettiques et des os. Elle est utilisée en complément du calcium pour prévenir l'ostéoporose chez les personnes âgées.

On sait aujourd'hui qu'environ 80% des personnes souffrent d'un déficit en vitamine D3.

Les besoins journaliers en vitamine D3 sont de 5 µg pour les enfants à partir de 4 ans jusqu'à l'âge adulte mais grimpe à 10 µg chez les enfants de moins de 3 ans, les femmes enceintes et les personnes âgées.

Chez les reptiles, la vitamine D3 permet notamment une assimilation optimale du calcium et la minéralisation des os.

Classiquement, les sources alimentaires contenant de la vitamine D3 proviennent essentiellement des poissons à travers notamment les huiles, le filet ou les foies de poisson.

Les poissons sont cependant une ressource en déclin, qui devient donc de plus en plus chère.

Pour ces raisons, il devient nécessaire de trouver des sources alternatives de vitamine D3 qui s'inscrivent dans une démarche durable et raisonnée.

Cette vitamine D3 dont les propriétés nutritionnelles sont décisives fait donc partie des vitamines liposolubles qu'on peut trouver chez certaines espèces de coléoptères comme les *Tenebrio molitor* et les *Alphitobius diaperinus.*

On sait déjà réaliser des poudres alimentaires à base de ces coléoptères.

L'étude « Klasing et al » (1999) montre que, chez les larves de *Tenebrio molitor,* la prise d'une alimentation sèche contenant 675 UI de vitamine D3 pour 1000 grammes de nourriture pendant 15 jours induisait une concentration en vitamine D3 de 139 UI.

Actuellement, le seul moyen connu d'augmenter le taux de vitamine D3 est donc d'ajouter dans l'alimentation de ces espèces une supplémentation en vitamine D3 sous forme de poudre.

Le Demandeur soumet que les concentrations en vitamine D3 pour 100 grammes de matière vivante ou sèche chez *Tenebrio molitor* ou *Alphitobius diaperinus* nourris avec une supplémentation en vitamine D3 sous forme de poudre reste cependant faible si on souhaite utiliser ces espèces comme complément alimentaire pour la vitamine D3 en nutrition humaine ou animale.

De telles poudres obtenues avec une supplémentation en vitamine D3 lors de l'élevage des coléoptères ne sont donc pas économiquement et nutritionnellement satisfaisantes.

### Résumé et objet de la présente invention

La présente invention vise à améliorer la situation actuelle décrite ci-dessus.

La présente invention vise plus particulièrement à remédier aux différents inconvénients mentionnés ci-dessus en proposant une solution pour augmenter significativement le taux de vitamine D3 chez le *Tenebrio molitor* ou *Alphitobius diaperinus* vivant et/ou dans la poudre obtenue après transformation sans passer par l'ajout croissant de vitamine D3 dans l'alimentation des insectes pendant le stade larvaire.

L'invention est exposée dans le jeu de revendications joint.

L'expression « stade larvaire » regroupe l'ensemble des stades avant le stade nymphal.

L'expression « stade nymphal » correspond quant à elle à la phase intermédiaire entre le dernier stade larvaire et le stade adulte.

Débuter la phase de traitement pendant la phase larvaire a montré des rendements intéressants.

On notera ici que l'émission de lumière dans le visible n'a aucune incidence sur la synthèse de la vitamine D3.

La distance entre la source UV et les larves de coléoptères est pertinente et permet avantageusement de limiter l'impact sur les larves de l'apport en chaleur inhérent à la source d'UV.

La puissance définie dans la revendication 1 est pertinente et permet avantageusement de limiter l'impact sur les larves de l'apport en chaleur inhérent à la source d'UV.

Les plages de traitement citées dans la revendication 4 induisent une synthèse plus importante de vitamine D3. Les larves sont maintenues dans l'obscurité totale lorsque le traitement UV est absent.

Ceci représente un bon rendement dans la synthèse de vitamine D3.

La plage de température de l'environnement citée dans la revendication 5 permet l'ajout d'une source UV sans que la chaleur additionnelle de cette source UV n'impacte la survie des larves de coléoptères.

La gestion contrôlée des paramètres ambiants permet d'obtenir un meilleur rendement dans la synthèse de la vitamine D3.

### Description détaillée portant sur différents exemples

Les exemples qui suivent sont fournis à titre de simple illustration de l'invention vis-à-vis de laquelle ils ne présentent aucun caractère limitatif.

Le procédé d'élevage qui va être décrit ici vise à mettre au point une technique pour augmenter significativement le taux de vitamine D3 chez le *Tenebrio molitor* ou *Alphitobius diaperinus* vivant et/ou dans la poudre obtenue associée après transformation sans passer par l'ajout croissant de vitamine D3 dans l'alimentation des insectes pendant le stade larvaire comme il est connu de le faire dans les techniques existantes.

Par souci de clarté et de concision, on entend par coléoptères dans la suite de la description les espèces suivantes : *Tenebrio molitor* et/ou *Alphitobius diaperinus.*

On sait qu'il est possible de faire varier les quantités des nutriments présents chez les coléoptères selon le stade pendant la croissance, le type d'alimentation, le mode de transformation et la qualité du conditionnement et du stockage.

Le concept sous-jacent à la présente invention est donc d'utiliser un traitement UV sur les coléoptères pour augmenter la teneur en vitamine D3, ceci sans supplémentation de vitamines D3 dans l'alimentation.

Le procédé d'élevage comprend ainsi plusieurs phases qui vont être décrites ici dans la suite de la description.

### - Phase initiale :

La formulation et les quantités de nourriture pour les larves de coléoptères sont contrôlées dès l'éclosion pour éviter tout biais dans les expérimentations.

Après éclosion, les larves sont maintenues dans des bacs plastiques à une température comprise entre 24 et 30°C, plus préférentiellement entre 26 et 28°C et une hygrométrie comprise entre 45 et 70% d'humidité relative, plus préférentiellement entre 55 et 65% d'humidité relative.

Avant la phase de traitement lumineux qui va suivre cette phase, les larves sont maintenues dans l'obscurité entre 12 et 22 heures par jour, plus préférentiellement entre 18 et 20h par jour.

Dans chaque bac, la densité de larves est optimisée pour obtenir un taux de mortalité proche de 0 sur le cycle de croissance compris entre 10 et 16 semaines, plus préférentiellement entre 11 et 13 semaines.

La nourriture fournie aux larves est 100 % végétal.

Les quantités de nourriture sont adaptées aux nombres de larves et à leur stade de développement.

Dans l'exemple décrit ici, on prévoit à partir de la sixième semaine de croissance de donner aux larves des légumes préalablement nettoyées dans des quantités adaptées aux nombres de larves et à leur stade de développement.

### - Phase de traitement lumineux ou traitement UV :

Dans les différents tests menés, on procède lors de cette phase à un traitement lumineux au cours duquel une source UV émet en direction des coléoptères des UVA et/ou des UVB.

Les différents tests montrent que l'intensité du traitement ultraviolet va directement impacter la concentration en vitamine D3 au des sein des larves.

Cette intensité en vitamine D3 va dépendre de plusieurs facteurs : la distance de la source UV, la puissance de la source UV, la fréquence du traitement, la durée du traitement et la période du traitement.

La distance de la source d'ultraviolet par rapport aux larves est placée entre 15 et 50 cm. Cette distance permettant de limiter l'impact sur les larves de l'apport en chaleur inhérent à la source d'UV.

La puissance de la source d'ultraviolet doit être comprise entre 20 et 50 Watts. Cette puissance permettant de limiter l'impact sur les larves de l'apport en chaleur inhérent à la source d'UV.

Sur une journée de traitement comportant 24 heures, la fréquence du traitement comprend des plages de traitement UV comprises entre 10h et 24h en continues ou cumulées sur les 24h. Ces plages de traitement induisent une synthèse plus importante de vitamine D3. Les larves sont maintenues dans l'obscurité totale lorsque le traitement UV est absent.

La durée du traitement est comprise entre 1 et 6 semaines, plus préférentiellement entre 2 et 4 semaines. Ces durées permettent d'obtenir des teneurs en vitamine D3 pertinentes pour l'utilisation de la poudre comme complément alimentaire.

Le traitement peut débuter entre la 6ème et la 12ème semaine de croissance des larves, plus préférentiellement entre la 9ème et 11ème semaine. Cette période correspond à des tailles de larves optimales.

Le spectre d'émission de la source d'ultraviolets comprend des rayonnements électromagnétiques avec des longueurs d'ondes entre 280 et 400 nm.

De préférence, la température et l'humidité relative pour chacune des phases ont été contrôlées afin qu'il n'y ait pas plus de 2°C et 10% HR de différence notamment entre les traitements avec et sans lampes (phase initiale et phase de traitement UV).

Les larves sont ensuite transformées comme décrit dans le paragraphe suivant.

### - Phase de transformation :

Entre la 6ème et la 14ème semaine de croissance, plus préférentiellement entre la 10ème et la 13ème semaine de croissance, les larves sont tamisées pour éliminer les excréments.

Les larves tamisées sont ensuite placées dans un bac plastique pour un jeun de 24 à 48 heures.

Après le jeun, les larves sont de nouveau tamisées pour enlever les excréments.

Les larves sont placées dans de l'eau entre 85°C et 100°C pour l'abattage pendant 1 à 4 minutes.

Lors de cette transformation, on prévoit en outre, juste avant l'abattage, une étape d'étourdissement entre 0 et 4°C pendant plusieurs minutes.

Après l'abattage, les larves sont placées à une température comprise entre 50 et 150°C pour une durée comprise entre 1h20 et 24h en fonction de la température utilisée. Les larves obtenues contiennent entre 2 et 15% d'eau, plus préférentiellement entre 3 et 8% d'eau et une activité de l'eau inférieure à 0.7

La réduction en poudre est ensuite obtenue par broyage.

### - Tests et résultats :

Pour mettre en évidence l'influence des UVA et UVB sur la concentration en vitamine D3 chez les larves de coléoptères, plusieurs tests ont été réalisées sur des larves âgées de 6 à 12 semaines et plus préférentiellement âgées de 9 à 11 semaines :
➢Test≪UV+/D3+≫:
   Un bac contenant 500 g de larves a été placé sous une lampe fluorescente UVA-UVB à 30 cm au-dessus du bac. Les larves ont été nourries depuis leur éclosion avec une alimentation supplémentée en vitamine D3 (2500 UI / 1000g).
➢Test≪UV+/D3-≫:
   Un bac contenant 500 g de larves a été placé sous une lampe fluorescente UVA-UVB à 30 cm au-dessus du bac. Les larves ont été nourries depuis leur éclosion avec une alimentation dépourvue de vitamine D3.
➢Test≪UV-/D3+≫:
   Un bac contenant 500 g de larves a été placé sous une lampe halogène de lumière visible (« Warm white », 2700K) à 30 cm au-dessus du bac ne contenant pas d'émission UV. Les larves ont été nourries depuis leur éclosion avec une alimentation supplémentée en vitamine D3 (2500 UI / 1000g).

Par lumière visible, on entend des rayonnements électromagnétiques dont la longueur d'onde est comprise entre 400nm et 700nm.

### - Test « Ctrl Dark » :

Un bac contenant 500 g a été placé à l'obscurité 24h/24h pendant une période similaire à celle des tests de traitement lumineux.

Les résultats de ces tests sont annexés à la présente description et montrent la teneur en vitamine D3.

Les analyses de quantification de la vitamine D3 ont été réalisées par un laboratoire indépendant certifié Cofrac. La quantification est réalisée par HPLC semi-préparative suivie d'une HPLC en phase inverse avec détecteur UV/DAD (265 nm).

Ces résultats mettent en évidence l'influence de l'UVB sur la concentration en vitamine D3.

Dans les tests « UV+ / D3+ » et « UV+ / D3- » réalisés ici, il est montré qu'un traitement lumineux à base d'UV permet de multiplier le taux de vitamine D3 jusqu'à 13.

On notera qu'une supplémentation en vitamine D3 pendant le traitement améliore les rendements. En revanche, sans supplémentation, la teneur en vitamine D3 est multiplié par 5, ce qui est très satisfaisant en termes de performance.

Les analyses démontrent que la concentration passe en effet de 2µg/100g à 26 µg/100g sous l'effet d'un traitement UV pendant le stade larvaire.

En revanche, un traitement lumineux à partir de lumière du spectre visible (400-700 nm) n'induit pas d'augmentation du taux de vitamine D3 chez les larves.

Les UV déclenchent donc la synthèse de vitamine D3 même en absence de supplémentation dans l'alimentation.

La présente invention porte donc notamment sur le procédé d'élevage de coléoptères pour la préparation d'une poudre de coléoptères tel que décrit ci-dessus.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

Il devra également être observé que les signes de références mis entre parenthèses dans les revendications qui suivent ne présentent en aucun cas un caractère limitatif ; ces signes ont pour seul but d'améliorer l'intelligibilité et la compréhension des revendications qui suivent ainsi que la portée de la protection recherchée.

| **Test** | **Concentration en vitamine D3 (µg/100g)** |
|---|---|
| UV+ / D3+ | 10 |
| UV+ / D3- | 26 |
| UV- / D3+ | 1.66 |
| Ctrl Dark | 2 |

## Revendications

1. Procédé d'élevage de coléoptères, de préférence des coléoptères sélectionnés parmi les espèces suivantes : *Tenebrio molitor, Alphitobus diaperinus,* pour la préparation d'une poudre de coléoptères comportant un pourcentage en poids de vitamine D3 supérieur ou égal à au moins 0.000010 %, ledit pourcentage en poids étant donné sur le poids total de poudre de coléoptères, **caractérisé en ce qu'**il comporte une phase de traitement lumineux au cours de laquelle une source UV émet des rayons ultraviolets du type UVB dont la longueur d'onde est comprise entre 280 nm et 320 nm, et/ou du type UVA dont la longueur d'onde est comprise entre 320 nm et 400 nm en direction des coléoptères, pendant le stade larvaire, **en ce que** la phase de traitement lumineux présente une durée de traitement comprise entre une et six semaines, de préférence entre deux et quatre semaines et **en ce que** ladite source UV présente une puissance de rayonnement comprise entre 13 et 125 Watts, de préférence entre 20 et 50 Watts.

2. Procédé selon la revendication 1, dans lequel la phase de traitement lumineux débute entre la sixième semaine et la douzième semaine de croissance des larves, de préférence entre la neuvième et la onzième semaine.

3. Procédé selon l'une quelconque des revendications précédentes, dans lequel, pendant la phase de traitement lumineux, la source UV est positionnée à une distance déterminée des larves de coléoptères comprise entre 2 à 250 cm, de préférence entre 10 et 100 cm, de préférence entre 15 et 50 cm.

4. Procédé selon l'une quelconque des revendications précédentes, dans lequel, pendant la phase de traitement lumineux, la source UV émet les rayons ultraviolets en direction des larves de coléoptères selon des plages de traitement comprises entre dix et vingt-quatre heures en continue ou en cumulée sur une période de vingt-quatre heures.

5. Procédé selon l'une quelconque des revendications précédentes, dans lequel les larves de coléoptères sont maintenues dans un environnement présentant :
- une température sensiblement constante comprise entre 24 et 30°C, de préférence entre 26 et 28°C ; et/ou
- une hygrométrie sensiblement constante comprise entre 45 et 70 % d'humidité relative, de préférence entre 55 et 65 %.

6. Procédé selon l'une quelconque des revendications précédentes, lequel comporte, préalablement à la phase de traitement lumineux, une phase initiale dite d'obscurité au cours de laquelle, après éclosion des larves de coléoptères, les larves sont maintenues dans l'obscurité entre douze et vingt-deux heures par jour, de préférence entre dix-huit et vingt heures par jour.

7. Procédé selon les revendications 5 et 6, dans lequel on contrôle la température et/ou l'hygrométrie de l'environnement des larves de coléoptères de manière à ce que, entre les phases d'obscurité et de traitement lumineux, la différence de températures est inférieure ou égale à 2 °C et/ou la différence d'hygrométries est inférieure ou égale à 10 % d'humidité relative.

8. Procédé selon l'une quelconque des revendications précédentes, dans lequel les larves de coléoptères sont nourries avec un régime alimentaire à base de végétaux et/ou de légumes.

## Patentansprüche

1. Verfahren zum Züchten von Käfern, vorzugsweise von Käfern, die aus den folgenden Spezies ausgewählt werden: *Tenebrio molitor, Alphitobus diaperimus,* zur Herstellung eines Käferpulvers, welches einen Gewichts-Prozentsatz an Vitamin D3 größer oder gleich von mindestens 0,000010% beinhaltet, wobei der Gewichts-Prozentsatz auf das Gesamtgewicht des Käferpulvers angegeben wird, **dadurch gekennzeichnet, dass** es eine Lichtbehandlungsphase beinhaltet, im Laufe derer eine UV-Quelle Ultraviolettstrahlen vom Typ UVB, deren Wellenlänge zwischen 280 nm und 320 nm liegt, und/oder vom Typ UVA, deren Wellenlänge zwischen 320 nm und 400 nm liegt, in Richtung der Käfer während des Larvenstadiums ausgibt, dadurch, dass die Lichtbehandlungsphase eine Behandlungsdauer aufweist, die zwischen einer und sechs Wochen, vorzugsweise zwischen zwei und vier Wochen liegt, und dadurch, dass die UV-Quelle eine Strahlungsleistung aufweist, die zwischen 13 und 125 Watt, vorzugsweise zwischen 20 und 50 Watt liegt.

2. Verfahren nach Anspruch 1, wobei die Lichtbehandlungsphase zwischen der sechsten Wachstumswoche und der zwölften Wachstumswoche der Larven, vorzugsweise zwischen der neunten und der elften Woche beginnt.

3. Verfahren nach einem der vorstehenden Ansprüche, wobei die UV-Quelle während der Lichtbehandlungsphase in einem bestimmten Abstand zu den Käferlarven positioniert wird, der zwischen 2 und 250 cm, vorzugsweise zwischen 10 und 100 cm, vorzugsweise zwischen 15 und 50 cm liegt.

4. Verfahren nach einem der vorstehenden Ansprüche, wobei die UV-Quelle während der Lichtbehandlungsphase Ultraviolett-Strahlen in Richtung der Käferlarven entsprechend den Behandlungsbereichen ausgibt, die zwischen 10 und 24 Stunden ununterbrochen oder kumuliert über einen Zeitraum von 24 Stunden liegen.

5. Verfahren nach einem der vorstehenden Ansprüche, wobei die Käferlarven in einer Umgebung gehalten werden, die Folgendes aufweist:
- eine im Wesentlichen konstante Temperatur, die zwischen 24 und 30°C, vorzugsweise zwischen 26 und 28°C liegt; und/oder
- eine im Wesentlichen konstante Luftfeuchtigkeit, die zwischen 45 und 70% an relativer Feuchtigkeit, vorzugsweise zwischen 55 und 65% liegt.

6. Verfahren nach einem der vorstehenden Ansprüche, welches vor der Lichtbehandlungsphase eine sogenannte ursprüngliche Dunkelheitsphase beinhaltet, im Laufe derer die Larven, nach dem Schlüpfen der Käferlarven, zwischen 12 und 24 Stunden pro Tag, vorzugsweise zwischen 18 und 20 Stunden pro Tag in der Dunkelheit gehalten werden.

7. Verfahren nach den Ansprüchen 5 und 6, wobei die Temperatur und/oder die Luftfeuchtigkeit der Umgebung der Käferlarven derart kontrolliert wird, dass der Temperaturunterschied zwischen den Phasen der Dunkelheit und der Lichtbehandlung kleiner oder gleich 2°C ist, und/oder der Luftfeuchtigkeitsunterschied kleiner oder gleich 10% an relativer Feuchtigkeit ist.

8. Verfahren nach einem der vorstehenden Ansprüche, wobei die Käferlarven mit einer Ernährung auf Basis von Pflanzen und/oder Gemüse gefüttert werden.

## Claims

1. A method for rearing beetles, preferably beetles selected from the following species: *Tenebrio molitor, Alphitobus diaperinus,* for preparing a beetle powder including a percentage by weight of vitamin D3 greater than or equal to at least 0.000010%, said percentage by weight being given on the total weight of beetle powder, **characterised in that** it includes a light treatment phase during which a UV source emits ultraviolet rays of the UVB type whose wavelength is comprised between 280 nm and 320 nm, and/or of the UVA type whose wavelength is comprised between 320 nm and 400 nm towards the beetles during the larval stage, **in that** the light treatment phase has a treatment duration comprised between one and six weeks, preferably between two and four weeks, and **in that** said UV source has a radiation power comprised 13 and 125 Watt, preferably between 20 and 50 Watts.

2. The method according to claim 1, wherein the light treatment stage starts between the sixth week and the twelfth week of growth of the larvae, preferably between the ninth and the eleventh week.

3. The method according to any one of the preceding claims, wherein, during the light treatment phase, the UV source is positioned at a defined distance from the beetle larvae comprised between 2 to 250 cm, preferably between 10 and 100 cm, preferably between 15 and 50 cm.

4. The method according to any one of the preceding claims, wherein, during the light treatment phase, the UV source emits the ultraviolet rays towards the beetle larvae according to treatment periods between ten hours and twenty-four hours continuously or cumulatively over a twenty-four hour period.

5. The method according to any one of the preceding claims, wherein the beetle larvae are kept in an environment having:
- a substantially constant temperature comprised between 24 and 30°C, preferably between 26 and 28°C; and/or
- a substantially constant hygrometry comprised between 45 and 70% relative humidity, preferably between 55 and 65%.

6. The method according to any one of the preceding claims, which includes, prior to the start of the light treatment phase, an initial so-called darkness phase during which, after hatching the beetle larvae, the larvae are kept in darkness between twelve and twenty-two hours per day, preferably between eighteen and twenty hours per day.

7. The method according to claims 5 and 6, wherein the temperature and/or hygrometry of the environment of the beetle larvae is controlled such that, between the darkness and light treatment phases, the difference in temperatures is less than or equal to 2°C and/or the difference in hygrometries is less than or equal to 10% relative humidity.

8. The method according to any one of the preceding claims, wherein the beetle larvae are fed with a diet based on plants and/or vegetables.
